# EUROPÄISCHE PATENTSCHRIFT

(11) **EP 0 287 956 B1**
(45) Veröffentlichungstag und Bekanntmachung des Hinweises auf die Patenterteilung: **24.11.1993**
(21) Anmeldenummer: 88105967.9
(22) Anmeldetag: 14.04.1988
(51) Int. Cl.: C07C 69/716, C07C 67/38, C07C 69/708, C07C 41/50, C07C 43/303, C07C 45/51, C07C 47/21

(54) **Verfahren zur Herstellung von 5-Formylvaleriansäureestern oder den entsprechenden Acetalen**
Process for the preparation of 5-formylevalerianic acid esters or the corresponding acetals
Procédé de préparation d'esters de l'acide 5-formylvalérianique ou des acétals correspondants

(30) Priorität: 23.04.1987 DE 3713554
(43) Veröffentlichungstag der Anmeldung: 26.10.1988
(73) Patentinhaber: BASF Aktiengesellschaft, 67063 Ludwigshafen (DE)
(72) Erfinder: Merger, Franz, Dr., D-6710 Frankenthal (DE); Bertleff, Werner, Dr., D-6806 Viernheim (DE); Liebe, Joerg, Dr., D-6710 Frankenthal (DE)

(56) Entgegenhaltungen:
- EP-A- 0 031 100
- DE-A- 2 517 447

## Beschreibung

Die vorliegende Erfindung betrifft ein Verfahren zur Herstellung von 5-Formylvaleriansäureestern oder den entsprechenden Acetalen, aus denen die Formylgruppe leicht freigesetzt werden kann.

5-Formylvaleriansäureester sind besonders als Vorstufe für die Herstellung von Caprolactam, Caprolacton oder Adipinsäure, aber auch als vielseitig verwendbare Zwischenprodukte von großem Interesse. So kann man sie in bekannter Weise durch aminierende Hydrierung zu 6-Aminocapronester und durch dessen Cyclisierung in Caprolactam überführen.

Gemäß dem Stand der Technik können 5-Formylvaleriansäureester durch Hydroformylierung von Pentensäureestern, wobei Pentensäureester mit endständiger Doppelbindung bevorzugt sind, erhalten werden. Der Zugang zu solchen Pent-4-ensäureestern ist jedoch nicht unproblematisch, wie aus der DE-OS 33 17 163 hervorgeht, da diese Produkte gegenüber den isomeren Pentensäureestern mit interner Doppelbindung thermodynamisch weniger stabil sind.

Der Erfindung lag nun die Aufgabe zugrunde, 5-Formylvaleriansäureester aus großtechnisch günstig zugänglichen Ausgangsstoffen in hohen Ausbeuten unter weitgehender Vermeidung von Nebenprodukten herzustellen.

Demgemäß wurde ein Verfahren zur Herstellung von 5-Formylvaleriansäureestern oder den entsprechenden Acetalen gefunden, das dadurch gekennzeichnet ist, daß man
a) Acetaldehydacetale der allgemeinen Formel I 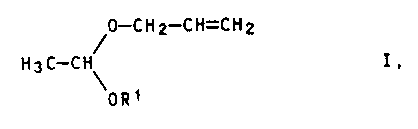 worin R¹ einen Alkyl- oder Alkenylrest mit 1 bis 4 C-Atomen bedeutet, bei 250 bis 400°C in der Gasphase an einem oberflächenaktiven Kontakt in Pent-4-enal II und R¹OH spaltet;
b) das Pent-4-enal nach Isolierung oder in situ mit Alkoholen R²OH in an sich bekannter Weise in das Pent-4-enalacetal der Formel III

   H₂C=CH-CH₂-CH₂-CH(OR²)₂ III,

   worin R² einen Alkyl- oder Alkenylrest mit 1 bis 4 C-Atomen bedeutet, überführt;
c) das Pent-4-enalacetal III bei 250 bis 400°C und unter einem Druck von 100 bis 400 bar mit Kohlenmonoxid und Alkoholen R³OH in Gegenwart von tertiären Stickstoffbasen und Cobaltcarbonylkatalysatoren zu 6,6-Dialkoxycapronsäureestern der allgemeinen Formel IV 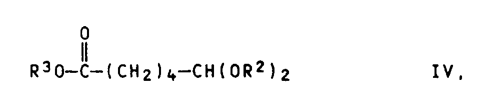 worin R² und R³ einen Alkylrest mit 1 bis 4 C-Atomen bedeutet, umsetzt und gegebenenfalls
d) den 5-Formylvaleriansäureester durch saure Hydrolyse der Acetalgruppe in IV freisetzt.

Nach der erfindungsgemäßen Kombination von Reaktionsschritten kann das gewünschte Produkt, ausgehend von Acetaldehyd, Allylalkohol, einem C₁-C₄-Alkohol und Kohlenmonoxid gemäß der folgenden Reaktionssequenz erhalten werden: 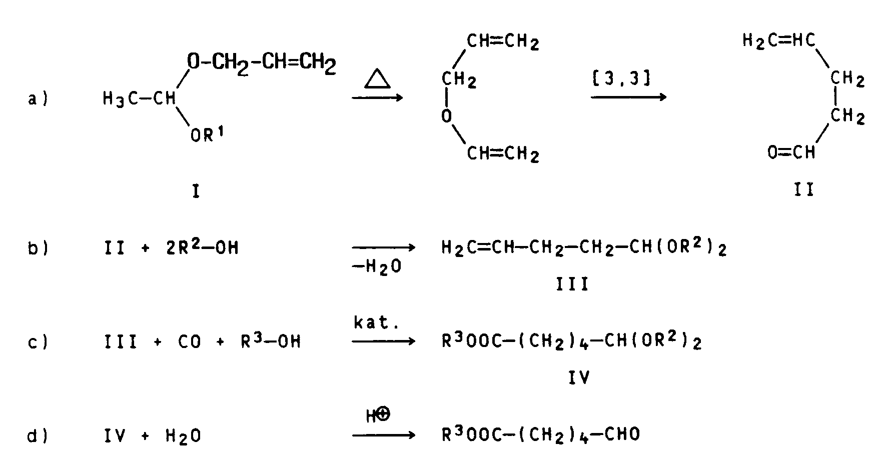
mit R¹ = C₁-C₄-Alkyl oder -Alkenyl und R² und R³ = C₁-C₄-Alkyl.

Stufe a) des Verfahrens ist in der DE-OS 25 17 447 beschrieben, wonach die Abspaltung eines Mols Alkohol R¹OH zum Allylvinylether und die nachfolgende Oxi-Cope-Umlagerung zum Pent-4-enal an oberflächenaktiven Stoffen wie z.B. Aktivkohle, entgaster Kohle, Aluminiumoxid, Titanoxid, Bimsstein oder Kieselgel, Eisenoxid, Zinkoxid oder mit einer dünnen Kohleschicht belegten, oxidischen Kontakten durchgeführt werden. Als besonders bevorzugtes Kontaktmaterial war Aluminiumoxid herausgestellt.

Verwendet man Acetaldehyddiallylacetal als Ausgangsstoff, so wird bei der Reaktion Allylalkohol abgespalten, der unter den Reaktionsbedingungen dazu neigt, sich in Propionaldehyd umzulagern. Diese Umlagerung konnte durch Verwendung von chemisch indifferenten Kontakten wie Kohle oder anderen Materialien, die mit 0,1 bis 4 % einer dünnen Schicht Kohle, etwa Ruß, belegt waren, zurückgedrängt werden.

Es wurde nun überraschend gefunden, daß Kieselgel, dotiert mit 1 bis 20 Gew.% Phosphorsäure und 0 bis 5, insbesondere 0,5 bis 4 Gew.% Alkalioxid, z.B. Lithium-, Natrium-, oder Kaliumoxid, ein besonders vorteilhaftes Kontaktmaterial darstellt, das zu hohen Umsätzen und Selektivitäten führt, ohne gleichzeitig die Isomerisierung des Allylalkohols zu Propionaldehyd zu begünstigen. Zweckmäßigerweise desaktiviert man den Kontakt mit einer dünnen Schicht Kohle, z.B. 0,1 bis 4 Gew.%, bezogen auf das Gesamtgewicht des Katalysators. Diese Belegung mit Kohle tritt im Laufe der Umsetzung in einer Induktionsperiode von ca. 1 bis 10h auch von selber ein, da bei der Thermolyse organischer Stoffe Kohlenstoff gebildet wird.

Die für die Reaktion notwendige Temperatur liegt bei 250 bis 400, bevorzugt 280 bis 350, insbesondere 300 bis 340°C und damit um ca. 50°C unter der nach der DE-OS 25 17 447 benötigten Temperatur.

Die Reaktion kann sowohl an einem fest angeordneten Kontakt als auch in der Wirbelschicht durchgeführt werden.

Beim Festbettverfahren verwendet man den Kontakt vorzugsweise in Form kugelförmiger, annähernd kugelförmiger oder strangförmiger Teilchen, deren Durchmesser 3 bis 8 mm beträgt. Um Nebenreaktionen weitgehend zu unterbinden, empfiehlt es sich, die mittleren Verweilzeiten der Acetale I am Kontakt nicht zu groß werden zu lassen. Bewährt haben sich Gasströmungsgeschwindigkeiten von 10 bis 300, vorzugsweise 20 bis 100 l pro Stunde, wobei das Gas nur aus Acetal I oder auch aus 30 bis 90 %igen Mischungen mit inerten Gasen wie Stickstoff oder Kohlendioxid bestehen kann.

Zur Erzielung eines 70- bis 100 %igen Umsatzes pro Durchgang, wie es sich als zweckmäßig erwiesen hat, und unter Berücksichtigung der angegebenen bevorzugten Strömungsgeschwindigkeiten, sind etwa 20 bis 100 cm hohe Kontaktschichten erforderlich. Der Querschnitt dieser Schichten ist der vorgegebenen Kapazität proportional. Pro Kilogramm I pro Stunde beträgt dieser Querschnitt etwa 10 bis 100 cm². Aus Gründen der Wärmeführung bevorzugt man an Stelle zusammenhängender Schichten mit großem Querschnitt mehrere Festbettsäulen mit je 10 bis 50 cm² Querschnitt.

Bei der Arbeitsweise in der Wirbelschicht gelten im Prinzip die gleichen Maßgaben. Unter Berücksichtigung der bevorzugten Teilchengrößen von 0,1 bis 0,8 mm, der genannten bevorzugten Strömungsgeschwindigkeiten und angegebenen bevorzugten Umsätze pro Durchgang, ergeben sich Wirbelbettschichten von bevorzugt 5 bis 40 cm Höhe. Bei Querschnitten von 10 bis 100 cm², wie sie in der Wirbelbettechnologie allgemein üblich sind, entspricht dies Leistungen von 0,04 bis 0,8 kg I pro Stunde.

Beide Verfahrensweisen, die man bei Normaldruck oder vorzugsweise bei einem Druck von 20 bis 400 mbar vornimmt, entsprechen den üblichen Methoden für katalytische Wirbelbett- bzw. Festbettverfahren, bieten keine besonderen Probleme und lassen sich an Hand einer Testreihe von Fall zu Fall leicht optimieren.

Nach beendeter Umsetzung am Kontakt werden die Gase fraktioniert kondensiert, wobei man das Pent-4-enal bei Umsätzen von 70 bis 100 % in etwa 90 bis 98 % Ausbeute erhält. Geht man von Acetaldehyddiallylacetal aus, so erhält man die entsprechende Menge Allylalkohol als weiteres Verfahrensprodukt, das sich durch azeotrope Destillation mit einem Hilfslösungsmittel wie z.B. Hexan abtrennen und zurückführen läßt. Bei Verwendung des oben beschriebenen Katalysators und einer bevorzugten Reaktionstemperatur von 300 bis 350°C lagert sich weniger als 5 % des Allylalkohols zu Propionaldehyd um.

Neben der Bedeutung Allyl, was wegen leichter Zugänglichkeit bevorzugt ist, kann R¹ auch für Alkylreste mit 1 bis 4 C-Atomen, z.B. für Methyl, Ethyl, Propyl oder Butyl sowie für den Homoallylrest stehen.

Stufe b) des Verfahrens, die Herstellung der Pent-4-enalacetale der Formel III, gelingt auf an sich bekannte Weise durch säurekatalysierte Acetalisierung mit Alkoholen der Formel R²OH, wobei R² eine Alkylgruppe mit 1 bis 4 Kohlenstoffatomen, z.B. eine Methyl, Ethyl, Propyl, Isopropyl- oder Butylgruppe darstellt. Bevorzugt steht R² für eine niedermolekulare Alkylgruppe, insbesondere für Methyl oder Ethyl.

Die Umsetzung kann bei einer Temperatur von 0 bis 120°C , vorzugsweise 20 bis 80°C , drucklos oder unter Druck, dikskontinuierlich oder kontinuierlich durchgeführt werden.

Man kann die Acetalisierung in einem unter den Reaktionsbedingungen inerten Lösungsmittel wie z.B. in Kohlenwasserstoffen, halogenierten Kohlenwasserstoffen oder Ethern vornehmen, wobei das Lösungsmittel vorteilhaft in einer Menge von 100 bis 1000 Gew.% bezogen auf II zugesetzt wird. In der bevorzugten Ausführungsform verwendet man einen Überschuß des für die Umsetzung benötigten Alkohols R²OH. Erforderlich für die Umsetzung sind pro Mol Pent-4-enal mindestens 2 mol Alkohol, bevorzugt ist ein Molverhältnis von Pent-4-enal zu Alkohol von 1:4 bis 1:50.

Die Umsetzung erfordert die Gegenwart von Säure als Katalysator. Die Menge an Katalysator kann vorteilhaft zwischen 0,001 und 0,2, insbesondere zwischen 0,01 und 0,15 Äquivalenten, pro Mol Pent-4-enal II, liegen. Es können anorganische oder organische Säuren verwendet werden, wie z.B. Schwefelsäure, Phosphorsäure, Chlorwasserstoff, aliphatische Carbonsäuren wie Ameisensäure, Chloressigsäure oder Sulfonsäuren wie Benzol- und p-Toluolsulfonsäure.

Wegen der problemlosen Aufarbeitung eignen sich saure Ionenaustauschharze besonders gut, da sie nach Beendigung der Reaktion nur abfiltriert zu werden brauchen und erneut ohne Ausbeuteverluste wiedereingesetzt werden können. Geeignet sind z.B. Duolite® C 265, Lewasorb® AC 10, Lewatit® SPC 118 oder Amberlyst® 15.

Die Reaktion kann vorteilhaft in der Weise durchgeführt werden, daß man ein Gemisch aus Pent-4-enal, Alkohol, Säure und gegebenenfalls Lösungsmittel auf die Reaktionstemperatur erhitzt und das freiwerdende Wasser chemisch z.B. durch Zugabe von Ameisensäuretrialkylestern oder von anderen wasserbindenden Stoffen oder physikalisch z.B. durch Abdestillieren mit Hilfe eines Azeotropbildners, der vorteilhaft gleichzeitig als Lösungsmittel eingesetzt werden kann, entfernt.

Die Aufarbeitung erfolgt vorzugsweise durch Destillation; evtl. noch unumgesetzte Ausgangsstoffe können rückgeführt werden.

Für die Herstellung des Pent-4-enalacetals III kann auch direkt das bei der Umsetzung der 1. Stufe erhaltene Produktgemisch, bestehend aus Pent-4-enal und gegebenenfalls Alkohol der allgemeinen Formel R¹-OH, eingesetzt werden. In diesem Fall sollte das Molverhältnis zwischen Pent-4-enal und Alkanol R²-OH zwischen 1:4 und 1:50, bevorzugt 1:10 und 1:50 liegen, um unerwünschte Acetalbildung aus Pent-4-enal und Alkohol R¹-OH zu vermeiden.

Die Abtrennung des gebildeten Pent-4-enalacetals der allgemeinen Formel III kann durch übliche Methoden, z.B. fraktionierende Destillation erfolgen.

Die Umsetzung der Stufe c), die Carbonylierung des Pent-4-enaldialkylacetals der Formel III zu 6,6-Dialkoxycapronsäurealkylestern der allgemeinen Formel IV, gelingt durch Umsetzung von III mit Kohlenmonoxid und Alkoholen der allgemeinen Formel R³-OH, wobei R³ die für R² genannte Bedeutung besitzt und gleich oder verschieden von R² ist, in Gegenwart von tertiären Stickstoffbasen und Kobaltcarbonylkatalysatoren, z.B. Dicobaltoctacarbonyl oder Cobaltverbindungen, die in der Lage sind, unter Kohlenmonoxid und Wasserstoff, der in Spuren im Gemisch immer vorhanden ist, HCo(CO)₄ zu bilden, z.B. binäre Cobaltsalze wie Halogenide, Carboxylate, Cobaltoxid, Cobaltmetall oder komplexe Cobaltverbindungen.

Geeignete tertiäre Stickstoffbasen sind vor allem N-heterocyclische Verbindungen wie das Pyridin und die damit verwandten Basen wie Chinolin, Isochinolin, sowie die alkyl-, aralkyl- und arylsubstituierten Derivate dieser Basen wie 2-Picolin, 3-Picolin, 4-Picolin, 2,3-Dimethylpyridin, 2,4-Dimethylpyridin, 3,5-Dimethylpyridin, 4-Benzylpyridin-4-Benzylpyridin und Vinylpyridin. Ferner sind auch entsprechende polymerfixierte Verbindungen geeignet, wie z.B. Polyvinylpyridin. Weiterhin kommen Trialkylamine wie Trimethylamin und Triethylamin sowie Dialkylarylamine wie N,N-Dimethylanilin und N,N-Diethylanilin in Betracht.

Die Carbonylierung von III kann vorteilhaft in Gegenwart eines Überschusses and Alkohol R³-OH bei Temperaturen von 140 bis 200, insbesondere 150 bis 180°C und unter Drucken von 100 bis 400 bar vorgenommen werden. Vorzugsweise beträgt die verwendete Menge an Alkoholen R³-OH 1,1 bis 4 mol je Mol III und an tertiärer Stickstoffbase 0,05 bis 0,4 mol je Mol III.

Die Reaktion kann diskontinuierlich oder auch kontinuierlich nach den dafür üblichen Techniken durchgeführt werden.

Nach dem Entspannen werden aus dem erhaltenen Reaktionsgemisch der überschüssige Alkohol und die freie tertiäre Stickstoffbase abdestilliert. Die chemisch gebundene tertiäre Stickstoffbase wird dabei nicht abdestilliert.

Das verbleibende, Katalysator, 6,6-Dialkoxycapronsäureester und Nebenprodukte enthaltende Reaktionsgemisch wird anschließend in üblicher Weise aufgearbeitet, z.B. durch weitere Destillation oder Extraktion, z.B. mit wäßrig-sauren Medien unter oxidierenden Bedingungen, wie es in DE 27 13 195 beschrieben ist. Der Katalysator und die tertiäre Stickstoffbase können rückgeführt und erneut eingesetzt werden. Nebenprodukte können durch fraktionierende Destillation abgetrennt werden.

Die Umsetzung der Stufe d), d.h. die Freisetzung der Formylgruppe aus der Acetalgruppierung in IV, kann in an sich bekannter Weise durchgeführt werden. Vorteilhaft kann man die Acetalspaltung bei Temperaturen von 0 bis 80, vorzugsweise 10 bis 60°C, drucklos oder unter Druck, diskontinuierlich oder kontinuierlich vornehmen. Man kann die Umsetzung ohne zusätzliches Lösungsmittel oder in Anwesenheit eines unter den Reaktionsbedingungen inerten Lösungsmittel durchführen. Vorzugsweise nimmt man das zur Reaktion benötigte Wasser gleichzeitig als Lösungsmittel. Bevorzugt wird Wasser im Molverhältnis 5:1 bis 100:1, bezogen auf den Capronsäurealkylester IV, eingesetzt.

Die Acetalspaltung erfordert die Gegenwart von Säure als Katalysator. Die Menge an Katalysator sollte zwischen 0,01 und 0,2 bevorzugt zwischen 0,01 und 0,15 mol, bezogen auf 1 Mol Capronsäurealkylester IV, liegen. Es können anorganische oder organische Säuren verwendet werden, wie z.B. Schwefelsäure, Phosphorsäure, Chlorwasserstoff, aliphatische Carbonsäuren wie Ameisensäure, Chloressigsäure oder Sulfonsäuren, wie Benzol- oder p-Toluolsulfonsäure. Wegen der problemlosen Aufarbeitung eignen sich saure Ionenaustauscherharze besonders gut, da sie nach Beendigung der Reaktion nur abfiltriert zu werden brauchen und erneut ohne Ausbeuteverlust wiedereingesetzt werden können. Geeignet sind z.B. Duolite® C 265, Lewasorb® AC 10, Lewatit® SPC 118 oder Amberlyst® 15

Zur Durchführung der Reaktion kann vorteilhaft ein Gemisch aus 6,6-Dialkoxycapronsäurealkylester, Säure, Wasser und gegebenenfalls Lösungsmittel auf Reaktionstemperatur erhitzt werden. Während der Reaktion wird das Gemisch je nach eingesetzter Menge an Wasser einphasig. Die für vollständigen Umsatz nötige Zeit hängt von den Reaktionsbedingungen, vor allem von der Temperatur ab und kann in einfachen Vorversuchen ermittelt werden.

Die Aufarbeitung kann in an sich bekannter Weise, z.B. durch kontinuierliche oder diskontinuierliche Extraktion mit einem inerten, mit Wasser nicht mischbaren Lösungsmittel, wie z.B. Methylenchlorid, Chloroform oder Essigsäureethylester und/oder durch Destillation vorgenommen werden. Der bei der Reaktion freigesetzte Alkohol R²-OH kann abgetrennt und in Vorstufen erneut eingesetzt werden. Die Reinigung der 5-Formylvaleriansäurealkylester erfolgt üblicherweise durch fraktionierende Destillation.

### Beispiel 1

### Herstellung des Pent-4-enals

Durch ein Wirbelbett von ca. 30 cm² Querschnitt und 18 cm Höhe, in welchem sich 350 ml feinkörniger Katalysator (mittlerer Teilchendurchmesser: 0,2 mm; Katalysator: Kieselgel, dotiert mit 11,5 Gew.-% Phosphorsäure, 3 Gew.-% Na₂O und oberflächlich belegt mit 2 Gew.-% Ruß) befindet, wurden bei 320 bis 340°C und unter einem Druck von 30 mbar pro Stunde 25 l (gerechnet unter Normalbedingungen) eines Gasgemisches aus 70 Vol.-% Acetaldehyddiallylacetal und 30 Vol.-% Stickstoff geleitet. Die übliche destillative Aufarbeitung lieferte Pent-4-enal mit einer Selektivität von 97 % bei einem Umsatz von 99 %. Die Selektivität beim Nebenprodukt Allylalkohol liegt bei 97 %; maximal 1,5 % sind zu Propionaldehyd umgelagert.

### Beispiel 2

### Vergleichsversuch zur Herstellung des Pent-4-enals gemäß DE-OS 25 17 447

Analog Beispiel 1, jedoch mit feinkörnigem Aluminiumoxid (mittlerer Teilchendurchmesser: 0,2 mm, oberflächlich belegt mit 2 Gew.-% Ruß) als Katalysator, wurden bei 380 bis 400°C unter einem Druck von 30 mbar pro Stunde 25 l (gerechnet unter Normalbedingungen) des im Beispiel 1 genannten Gasgemisches geleitet. Bei einem Umsatz von 79 % lag die Selektivität bei 91 %; 35 % des als Nebenprodukt anfallenden Allylalkohols waren zu Propionaldehyd umgelagert.

### Beispiel 3

### Vergleichsversuch zur Herstellung des Pent-4-enals gemäß DE-OS 25 17 447

Analog Beispiel 1, jedoch mit einem Aluminiumoxid, auf das etwas Ruß (ca. 2 Gew.-%) niedergeschlagen war, wurde bei geringerem Umsatz (48 %) als in Beispiel 2 eine höhere Selektivität an Pent-4-enal (94 %) erreicht, dennoch waren 8 % des entstandenen Allylalkohols zu Propionaldehyd umgelagert.

### Beispiel 4

### Herstellung des Pent-4-enaldimethylacetals

Zu einem Gemisch aus 64 g Methanol, 53 g Trimethylorthoformiat und 5 g saurem Ionenaustauscher (Amberlyst® 15) wurden 42 g Pent-4-enal getropft. Nach 6 h bei 25°C wurde destillativ aufgearbeitet. Man erhielt 57,8 g Pent-4-enaldimethylacetal, entsprechend einer Selektivität von 95 % bei einem Umsatz von 94 %.

### Beispiel 5

### Herstellung des 6,6-Dimethoxycapronsäuremethylesters

240 g Pent-4-enaldimethylacetal, 76,8 g Methanol, 9.8 g Dicobaltoctacarbonyl und 35,3 g 3-Picolin wurden in einem 2,5 l Magnethubautoklaven 6 Stunden bei einem Kohlenmonoxiddruck von 150 bis 160 bar auf 165°C erhitzt. Nach dem Abkühlen und Entspannen wurde der Austrag gaschromatographisch analysiert. Bei einem Umsatz von 93,4 % erhielt man 6,6-Dimethoxycapronsäuremethylester mit einer Selektivität von 76 %. Durch fraktionierende Destillation konnte 6,6-Dimethoxycapronsäuremethylester isoliert werden.

### Beispiel 6

### Herstellung des 5-Formylvaleriansäuremethylesters

47,5 g 6,6-Dimethoxycapronsäuremethylester wurden mit 45 g Wasser und 2 g Amberlyst® 15 1 Stunde bei 40°C gerührt. Nach Abfiltrieren des sauren Ionenaustauschers wurde gaschromatographisch analysiert: Bei einem Umsatz von 93 % erhielt man 5-Formylvalerianester mit einer Selektivität von 96 % d. Th. Durch Extraktion mit einem mit Wasser nicht mischbaren Lösungsmittel, z.B. Methylenchlorid, läßt sich der 5-Formylvalerianester isolieren.

## Patentansprüche

1. Verfahren zur Herstellung von 5-Formylvaleriansäureestern oder den entsprechenden Acetalen, dadurch gekennzeichnet, daß man
a) Acetaldehydacetale der allgemeinen Formel I 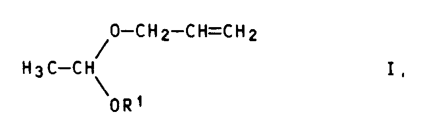 worin R¹ einen Alkyl- oder Alkenylrest mit 1 bis 4 C-Atomen bedeutet, bei 250 bis 400°C in der Gasphase an einem oberflächenaktiven Kontakt in Pent-4-enal II und R¹OH spaltet;
b) das Pent-4-enal nach Isolierung oder in situ mit Alkoholen R²OH in an sich bekannter Weise in das Pent-4-enalacetal der Formel III
H₂C=CH-CH₂-CH₂-CH(OR²)₂ III,
worin R² einen Alkyl- oder Alkenylrest mit 1 bis 4 C-Atomen bedeutet, überführt;
c) das Pent-4-enalacetal III bei 250 bis 400°C und unter einem Druck von 100 bis 400 bar mit Kohlenmonoxid und Alkoholen R³OH in Gegenwart von tertiären Stickstoffbasen und Cobaltcarbonylkatalysatoren zu 6,6-Dialkoxycapronsäureestern der allgemeinen Formel IV 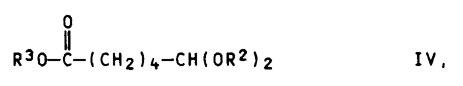 worin R² und R³ einen Alkylrest mit 1 bis 4 C-Atomen bedeuten, umsetzt und gegebenenfalls
d) den 5-Formylvaleriansäureester durch saure Hydrolyse der Acetalgruppe in IV freisetzt.

2. Verfahren nach Anspruch 1, dadurch gekennzeichnet, daß man als oberflächenaktiven Kontakt Kieselgel verwendet, das mit 1 bis 20 Gew.% Phosphorsäure und 0 bis 5 Gew.% Alkalioxid dotiert und gegebenenfalls mit 0,1 bis 4 % Kohle belegt ist.

3. Verfahren nach den Ansprüchen 1 und 2, dadurch gekennzeichnet, daß man als Acetal I Acetaldehyddiallylacetal verwendet.

4. Verfahren nach den Ansprüchen 1 bis 3, dadurch gekennzeichnet, daß man die Spaltung des Acetaldehydacetals I bei 300 bis 350°C vornimmt.

5. Verfahren nach den Ansprüchen 1 bis 4, dadurch gekennzeichnet, daß man die Spaltung bei einem Druck von 20 bis 400 mbar vornimmt.

6. Verfahren nach Anspruch 1, dadurch gekennzeichnet, daß man die Umsetzung von III mit Kohlenmonoxid bei einer Temperatur von 140 bis 200°C vornimmt.

7. Verfahren nach Anspruch 1, dadurch gekennzeichnet, daß man pro Mol III 1,1 bis 4 mol R³OH und 0,01 bis 0,1 mol des Cobaltkatalysators verwendet.

8. Verfahren nach den Ansprüchen 1 und 7, dadurch gekennzeichnet, daß man als Cobaltkatalysator Dicobaltoctacarbonyl oder HCo(CO)₄ verwendet.

9. Verfahren nach Anspruch 1, dadurch gekennzeichnet, daß man als tertiäre Stickstoffbase N-heterocyclische Verbindungen verwendet.

10. Verfahren nach Anspruch 1, dadurch gekennzeichnet, daR R² und R³ für eine Methylgruppe stehen.

## Claims

1. A process for preparing 5-formylvaleric esters or the corresponding acetals, characterised in that
a) acetaldehyde acetals of the general formula I 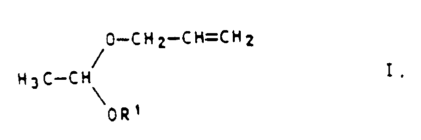 where R¹ is an alkyl or alkenyl radical of from 1 to 4 carbon atoms, are cleaved at from 250 to 400°C in the gas phase over a surface-active catalyst into 4-pentenal II and R¹OH,
b) the 4-pentenal is, after isolation or in situ, converted with alcohols R²OH in a conventional manner into the 4-pentenal acetal of the formula III
H₂C=CH-CH₂-CH₂-CH(OR²)₂ III
where R² is an alkyl or alkenyl radical of from 1 to 4 carbon atoms,
c) the 4-pentenal acetal III is reacted at from 250 to 400°C under a pressure of from 100 to 400 bar with carbon monoxide and alcohols R³OH in the presence of tertiary nitrogen bases and cobalt carbonyl catalysts to form 6,6-dialkoxycaproic esters of the general formula IV 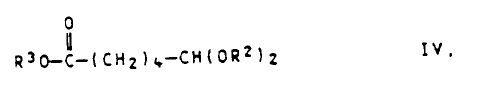 where R² and R³ are each an alkyl radical having from 1 to 4 carbon atoms, and if desired
d) the 5-formylvaleric ester is liberated by acid hydrolysis of the acetal group of IV.

2. A process as claimed in claim 1, characterised in that the surface-active catalyst is silica gel which has been doped with from 1 to 20 % by weight of phosphoric acid and from 0 to 5 % by weight of an alkali metal oxide and optionally coated with from 0.1 to 4 % of carbon.

3. A process as claimed in claim 1 or 2, characterized in that the acetal I is acetaldehyde diallyl acetal.

4. A process as claimed in any of claims 1 to 3, characterised in that the cleavage of the acetaldehyde acetal I is carried out at from 300 to 350°C.

5. A process as claimed in any of claims 1 to 4, characterised in that the cleavage is carried out at a pressure of from 20 to 400 mbar.

6. A process as claimed in claim 1, characterised in that the reaction of III with carbon monoxide is carried out at from 140 to 200°C.

7. A process as claimed in claim 1, characterised in that, per mole of III, from 1.1 to 4 mole of R³OH and from 0.01 to 0.1 mole of the cobalt catalyst are used.

8. A process as claimed in claim 1 or 7, characterised in that the cobalt catalyst is dicobalt octacarbonyl or HCo(CO)₄.

9. A process as claimed in claim 1, characterised in that the tertiary nitrogen base is an N-heterocyclic compound.

10. A process as claimed in claim 1, characterised in that R² and R³ are each methyl.

## Revendications

1. Procédé de fabrication d'esters de l'acide 5-formylvalérianique, ou des acétals correspondants, caractérisé en ce que
a) on scinde des acétaldéhydeacétals de la formule générale I 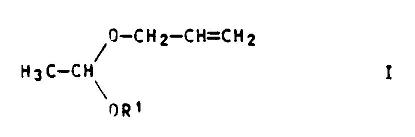 dans laquelle R¹ représente un radical alkyle ou alcényle qui comporte de 1 à 4 atomes de carbone, à 250-400°C, en phase gazeuse et sur un catalyseur de contact à surface active, en pent-4-énal II et R¹OH,
b) on convertit le pent-4-énal, après isolement ou in situ, avec des alcools R²OH, de manière en soi connue, en le pent-4-énalacétal de la formule III
H₂C=CH-CH₂-CH₂-CH(OR²)₂ III,
dans laquelle R² représente un radical alkyle ou alcényle, qui comporte de 1 à 4 atomes de carbone.
c) on convertit le pent-4-énalacétal III à 250-400°C et sous une pression de 100 à 400 bars avec du monoxyde de carbone et des alcools R³OH, en présence de bases à azote tertiaire et de catalyseurs du type cobaltcarbonyle, en esters d'acides 6,6-dialcoxycaproïques de la formule générale IV 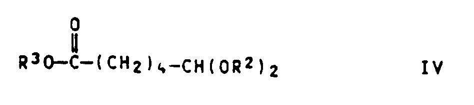 dans laquelle R² et R³ représentent chacun un radical alkyle qui comporte de 1 à 4 atomes de carbone et, éventuellement,
d) on libère l'ester de l'acide 5-formylvalérianique par hydrolyse acide du groupe acétal dans le composé IV.

2. Procédé suivant la revendication 1, caractérisé en ce que, à titre de catalyseur de contact à surface active, on utilise du gel de silice dopé de 1 à 20% en poids d'acide phosphorique et de 0 à 5% en poids d'un oxyde de métal alcalin et qui est éventuellement garni de 0,1 à 4% de charbon.

3. Procédé suivant les revendications 1 et 2, caractérisé en ce que l'on utilise l'acétaldéhydediallylacétal à titre d'acétal I.

4. Procédé suivant les revendications 1 à 3, caractérisé en ce que l'on entreprend la scission de l'acétaldéhydeacétal I à 300-350°C.

5. Procédé suivant les revendications 1 à 4, caractérisé en ce que l'on entreprend la scission sous une pression de 20 à 400 mbars.

6. Procédé suivant la revendication 1, caractérisé en ce que l'on entreprend la conversion de III avec du monoxyde de carbone à une température de 140 à 200°C.

7. Procédé suivant la revendication 1, caractérisé en ce que, par mole de composé III, on utilise 1,1 à 4 moles de R³OH et 0,01 à 0,1 mole du catalyseur au cobalt.

8. Procédé suivant les revendications 1 et 7, caractérisé en ce que l'on utilise du dicobaltoctacarbonyle ou Hco(CO)₄ à titre de catalyseur au cobalt.

9. Procédé suivant la revendication 1, caractérisé en ce que l'on utilise des composés N-hétérocycliques à titre de bases à azote tertiaire.

10. Procédé suivant la revendication 1, caractérisé en ce que R² et R³ représentent chacun le radical méthyle.
